Europäisches Patentamt

European Patent Office

Office européen des brevets

(19)

(11) Publication number: **0 426 154 A2**

# EUROPEAN PATENT APPLICATION

(21) Application number: 90120906.4

(22) Date of filing: 31.10.90

(51) Int. Cl.⁵: **A61K 6/033**, A61L 27/00

(30) Priority: 31.10.89 JP 284209/89

(43) Date of publication of application:
08.05.91 Bulletin 91/19

(84) Designated Contracting States:
**DE FR GB NL SE**

(71) Applicant: **ASAHI KOGAKU KOGYO
KABUSHIKI KAISHA
36-9, Maeno-cho 2-chome Itabashi-ku
Tokyo(JP)**

(72) Inventor: **Sumita, Masaya, c/o Asahi Kogaku
Kogyo Kabushiki Kaisha, 36-9, Maeono-cho
2-chome
Itabashi-ku, Tokyo(JP)**
Inventor: **Tamura, Naoji, c/o Asahi Kogaku
Kogyo Kabushiki Kaisha, 36-9, Maeono-cho
2-chome
Itabashi-ku, Tokyo(JP)**
Inventor: **Yokoyama, Atsurou
Nishi 3-chome, Kitajurokujo
Kita-ku, Sapporo-shi, Hokkaido(JP)**

(74) Representative: **Hansen, Bernd, Dr.
Dipl.-Chem. et al
Hoffmann, Eitle & Partner Patent- und
Rechtsanwälte Arabellastrasse 4
W-8000 München 81(DE)**

(54) Calcium phosphate biomaterial and process for production thereof.

(57) A biomaterial is disclosed, which biomaterial comprises calcium oxide and a apatite wherein the calcium oxide is substantially in phase separated form in the apatite.

EP 0 426 154 A2

## CALCIUM PHOSPHATE BIOMATERIAL AND PROCESS FOR PRODUCTION THEREOF

### FIELD OF THE INVENTION

The present invention relates to a biomaterial for dental and medical applications and a method for the production thereof.

### BACKGROUND OF THE INVENTION

Due to their superior biocompatibility and osteo-conductivity, hydroxyapatites have widely been studied for their dental and medical applications, and several types of these materials have already been put into practice. However, in order to promote bone regeneration to heal up the affected part as soon as possible, a long standing need exists to develop materials which have a higher rate of neogenetic bone formation that is faster than that of conventional biomaterials.

### SUMMARY OF THE INVENTION

An object of the present invention is therefore to provide a calcium phosphate biomaterial comprising apatite as a main component and having a high neogenetic bone formation rate, as well as a method for effectively producing the same.

Other objects and effects of the present invention will be apparent from the following description.

The present invention provides a biomaterial comprising calcium oxide and an apatite, wherein the calcium oxide is substantially in phase separated form in the apatite.

The present invention also provides a process for producing a biomaterial, comprising the step of: sintering an calcium-excess apatite at a temperature of at least 900° C. In another embodiment, a process is provided, which comprises the steps of: (A) mixing calcium oxide, or a compound capable of forming calcium oxide, with pure apatite powder to form a mixture; and (B) sintering the mixture at a temperature of at least 900° C.

### DETAILED DESCRIPTION OF THE INVENTION

Previously, calcium oxide has been considered unsuitable for medical and dental applications because calcium oxide will react with water in a living body to form calcium hydroxide, when it exists in a hydroxyapatite, as described, e.g., in JP-A-55-130854. (The term "JP-A" as used herein means an "unexamined published Japanese patent application"). However, unexpectedly and as taught away from by the related art, the present inventors have discovered that a material in which calcium oxide is contained in a sintered hydroxyapatite in the phase-separated form is medically and dentally suitable. Such hydroxyapatites of the present invention have been also advantageously discovered to have a higher rate of neogenetic bone formation than that of pure apatites.

The biomaterials of the present invention preferably comprises calcium oxide in an amount of from 2 to 60% by weight, more preferably from 2 to 20% by weight, based on the total amount of the biomaterial. If the calcium oxide content is less than 2% by weight, there is a little improvement of the rate of neogenetic bone formation. If the calcium oxide content exceeds about 60% by weight, the sintering ability tends to be unsuitably decreased.

In biomaterials of the present invention, which comprise apatite as a main component, as described above, the apatite is not limited to hydroxyapatite, but may be fluorinated apatite, which has more excellent acid resistance than that of hydroxyapatite. The apatite used in the present invention is preferably a sintered apatite. Further, if it is desired to impart intracorporeal absorbability to such a biomaterial, the biomaterial can further comprise tricalcium phosphate.

The present invention further provides a process for producing a biomaterial, as described herein. The process for producing the biomaterial of the present invention are characterized by sintering a calcium-excess apatite at a temperature of at least about 900° C to cause calcium oxide to be phase-separated in the hydroxyapatite or other apatite such as fluorinated apatite. The sintering temperature is preferably about 1,500° C or less, more preferably 1,300° C or less. The sintering time is generally 1 hour or more.

The term "calcium-excess apatite" as used herein refer to an apatite having a Ca/P molar ratio exceeding 10/6, which can be obtained, e.g., by the following methods.

When a calcium compound is reacted with a phosphate compound using, e.g., a conventional wet synthesis method, either (1) the calcium compound is added in a larger amount than the stoichiometrical amount for forming the pure hydroxyapatite, or (2) the phosphate compound is added in a smaller amount than the stoichiometrical amount for forming the pure hydroxyapatite.

When such an apatite is provided by the method (1) or (2) above, the resulting apatite having large calcium content is sintered at a temperature at least 900°C, and thus calcium oxide will be phase-separated. In such a process of the present invention, the amount of calcium oxide to be phase-separated may be regulated by controlling the Ca/P molar ratio or by the sintering temperature. That is, a larger Ca/P molar ratio and/or a higher sintering temperature provides a larger amount of calcium oxide thus phase-separated.

The biomaterials of the present invention can also be produced by mixing powdery calcium oxide, or a powdery compound capable of forming calcium oxide by heating, with a pure apatite, such as hydroxyapatite, and sintering the mixture at a temperature of at least 900°C. The sintering temperature is preferably about 1,500°C or less, more preferably 1,300°C or less. The sintering time is generally 1 hour or more. Examples of the compounds capable of forming calcium oxide by heating include calcium carbonate, calcium acetate, and calcium hydroxide.

In the process of the present invention, calcium oxide is preferably phase-separated so that from 2 to 60% by weight, preferably from 2 to 20% by weight, of calcium oxide is provided in the resulting sintered apatite.

The biomaterial of the present invention may be porous or non-porous, but is preferably porous in view of the biocompatibility. As the method for producing a porous sintered body, any conventional methods may be employed.

The biomaterials of the present invention have a higher rate of a neogenetic bone formation as compared to conventional biomaterials having pure apatite, and, thus, the present invention attains accelerated bone regeneration and accelerated healing of the affected part without any harmful affects to the living body.

The present invention will be described in more detail by reference to the following example and comparative example, but the present invention is not construed as being limited thereto.

EXAMPLE

A conventional wet method was used for synthesizing hydroxyapatite from a calcium hydroxide slurry and an aqueous phosphoric acid solution. After the reaction was carried out using an amount of the aqueous phosphoric acid solution less than the stoichiometric amount for producing hydroxyapatite, calcium-excess apatite powder was obtained by spray drying the reaction product into the powder form. The Ca/P molar ratio of the calcium-excess apatite powder was 1.78

The calcium-excess apatite powder obtained was resuspended in water so that the solid content was 50% by weight. The resulting slurry was impregnated in a polyurethane foam, and then the excess slurry was squeezed out. Then it was dried in an electric drier for 24 hours at 100°C. The polyurethane foam having the apatite coated thereon was obtained. This polyurethane foam was heated in a degreasing furnace at 500°C for 8 hours to make the polyurethane form disappear, and then heated in an electric furnace at 1,200°C for 4 hours to obtain a porous sintered body of calcium-excess apatite. This apatite was found to comprise about 3.8% by weight calcium oxide in the phase-separated form.

The resulting porous sintered body was processed into a pellet having a 5 mm-diameter and a 2 mm-thickness, and was used as samples for animal experiments. The samples were embedded in rat subperiosteal parietal region for one, three, four, and eight weeks, respectively. After sacrificing the animals, a decalcificated paraffin specimens of the implanted parts were prepared and were subjected to the H.E. staining. The samples were examined histologically, and the following results were obtained.

After one week-embedding, although there existed blood clots around the porous sintered body of calcium-excess apatite (hereinafter referred to as a "pellet") and in the pores thereof, it was observed that mesenchyma cells existed in part of the pores. Some neogenetic bone could be found in the mother bone around the pellet, but could not be found in contact with the pellet. After three week-embedding, the mesenchyma cells inside of the pores were increased, and neogenetic bones was in contact with the pellet. Neogenetic bone around the pellet and in the pores further increased from 4 to 8 weeks.

COMPARATIVE EXAMPLE

The same procedure as in the above Example was performed, except that a pure apatite was used in place of the calcium-excess sintered apatite, and the following results were obtained.

No neogenetic bone was found in contact with the embedded pellet, even after three week-embedding, and neogenetic bone in contact with the pellet were only partly found after four week-embedding. From this time to 8 week-embedding, neogenetic bone around the pellet and in the pores had a tendency to increase, but was substantially less than the increase in neogenetic bone found in the Example of the present invention.

While the invention has been described in detail and with reference to specific examples thereof, it will be apparent to one skilled in the art that various changes and modifications can be made therein without departing from the spirit and scope thereof.

## Claims

1. A biomaterial comprising calcium oxide and a apatite, wherein said calcium oxide is substantially in phase separated form in said apatite.

2. A biomaterial as claimed in claim 1, wherein said biomaterial comprises calcium oxide in an amount of from 2 to 60% by weight based on the total amount of said biomaterial.

3. A biomaterial as claimed in claim 2, wherein said biomaterial comprises calcium oxide in an amount of from 2 to 20% by weight based on the total amount of said biomaterial.

4. A biomaterial as claimed in claim 1, wherein said apatite is a sintered apatite.

5. A process for producing a biomaterial, said process comprising the step of: sintering an calcium-excess apatite at a temperature of at least 900°C.

6. A process for producing a biomaterial, said process comprising the steps of: (A) mixing calcium oxide, or a compound capable of forming calcium oxide, with pure apatite powder to form a mixture; and (B) sintering said mixture at a temperature of at least 900°C.

7. A process as claimed in claim 6, wherein said compound capable of forming calcium oxide is selected from the group consisting of calcium carbonate, calcium acetate, and calcium hydroxide.